# EUROPEAN PATENT APPLICATION

(11) **EP 3 002 004 A1**
(43) Date of publication of application: **06.04.2016**
(21) Application number: 15189356.7
(22) Date of filing: 24.10.2003
(51) Int. Cl.: A61K 9/06, A01N 25/00, A61K 31/05, A61K 8/63, A61K 8/69, A61K 8/34, A61K 8/67, A61K 9/107, A61K 31/203, A61K 31/58, A61Q 19/02

(54) **TOPICAL SKIN CARE COMPOSITION**

(30) Priority: 25.10.2002 US 280483
(62) Divisional of application: 03776536.9
(71) Applicant: Galderma S.A., 6330 Cham (CH)
(72) Inventor: PUGLIA, Nancy, Sanford, FL Florida 32773 (US); RAMIREZ, Rosario, Sanford, FL Florida 32773 (US); ROTH, Jerry, Sanford, FL Florida 32773 (US)
(74) Representative: Harrison, Susan Joan

(57) **Abstract**

A cream base for the topical application of skin care therapeutics and a process for making the cream base. In one embodiment, the therapeutic is tretinoin, hydroquinone and fluocinolone acetonide for the treatment of hyperpigmented skin conditions, such as melasma.

## Description

### FIELD OF THE INVENTION

The invention relates generally to a method of making a medicated skin treating composition.

### BACKGROUND OF THE INVENTION

Melasma or chloasma is a common pigmentary condition that affects primarily women in their reproductive years. Dark, mottled (hyperpigmented) patches appear on the face and neck, especially on the cheeks and forehead. Melasma is usually triggered by hormonal activity that is the result of pregnancy or birth control pills. Thus, the condition is known as the "mask of pregnancy." The condition occurs when excess melanin is deposited in the cells of the epidermis and dermis. Melasma can persist for long periods of time and often recurs with subsequent pregnancies. The condition is less common among men, who account for about 10% of all cases.

Standard therapy involves depigmenting, or bleaching, the affected areas of the skin, the use of sunscreens, and avoidance of sunlight. Hydroquinone is the most popular topical depigmenting agent. Concentrations of 5%-10% hydroquinone are very effective, but can be irritating. The chemical stability of hydroquinone formulations is important because hydroquinone is easily oxidized and loses potency. The most commonly used agent usually involves a 16-week to 20-week course of therapy, and some therapies can take longer. Tretinoin (Retin-A) is another widely used therapy for melasma.

Nevertheless, there remains a need in the art for a therapeutic approach that would contain several medicines for the treatment of melasma in a single composition. Moreover, it would be useful to have a therapeutic carrier, such as a cream, that would facilitate the penetration of the medicaments into the skin.

U.S. Pat. No. 5,538,737 discloses a method of making a water-in-oil emulsion containing a pharmaceutically acceptable salt of an H₂-antagonist. The steps include dissolving the pharmaceutically acceptable salt in an aqueous medium to form a water portion; combining the water portion with an oil portion, comprising an edible oil comprising an ester or mixed ester of glycerol and an emulsifying agent to form a water portion and oil portion matrix; then emulsifying the matrix to form the water-in-oil emulsion.

U.S. Pat. No. 5,656,672 discloses a process for preparing a water-in-oil emulsion with retinal as the active ingredient. The emulsion contains an oil phase including at least one organic solvent for retinal (such as aliphatic fatty alcohols) and optional lipophilic additives; an aqueous phase containing water and optional hydrophilic additives; and an agent for emulsifying the aqueous phase in the oil phase. The oil phase and the aqueous phase are independently prepared, and the aqueous phase is incorporated into the oil phase, with subsequent addition of a phase-containing retinol and its solvent.

U.S. Pat. No. 5,660,837 discloses a process for the preparation of a pharmaceutical formulation in the form of an oil-in-water emulsion. The steps of the process include of adding the emulsion-stabilizing surface active drug and an optimal conventional surfactant to a two-phase, oil-water system at room temperature; allowing the emulsion-stabilizing surface active drug to equilibrate at an interface; adding an agent giving isotonicity to the final formulation; and homogenizing by high pressure technique.

U.S. Pat. No. 5,976,555 discloses skin care compositions. An oil-in-water emulsion base contains retinoids; cetearyl alcohol and cetearyl glucoside or a mixture of a polyethylene glycol ethers of stearyl alcohol; cetyl alcohol, stearyl alcohol and mixtures thereof; a light, dry absorbable oil; and substantive, emollient oils or waxes.

U.S. Pat. No. 6,080,393 discloses a skin care composition comprising an oil-in-water emulsion with a therapeutically effective amount of a retinoid; wherein the oil phase comprising one or more oils, and an effective amount of at least one oil-soluble antioxidant; and wherein the composition comprises a corticosteroid.

Nevertheless, there remains a need in the art for a method of making a smoother cream base for the application of therapeutic agents for the treatment of melasma, which will facilitate the penetration of the medicaments into the skin.

### SUMMARY OF THE INVENTION

The invention provides a cream base for the topical application of skin care therapeutics and a process for making the cream base.

The process for making the cream base entails (a) mixing the hydrophilic compounds with water to form an aqueous phase; (b) mixing the hydrophobic compounds to form a hydrophobic (non-aqueous or wax) phase; then (c) mixing the hydrophilic and hydrophobic phases with one another to form a biphasic mixture; and finally (d) adding an emulsifier to the biphasic mixture to form the emulsion. By mixing the emulsifier after the aqueous and non-aqueous phases have been mixed, the result is a smoother-textured cream that disappears on application to skin, as compared to creams made by processes where the emulsifier is added to the aqueous or non-aqueous phases earlier in the process. Because the emulsifier is added as the final step, less wax is needed in making the cream, resulting in a "thinner" hydrophilic cream that disappears faster when applied to the skin, as compared to creams made by processes where the emulsifier is added to the aqueous or non-aqueous phases earlier in the process.

The cream base made by the method of interest can be a carrier for any of a variety of pharmaceutically active agents for dermatologic use. For example, anti-acne, anti-cancer, antibiotic, anti-inflammatory, hormone, anti-fungal and analgesic active agents can be incorporated into a cream base of interest.

In a specific embodiment, a cream base of interest comprises a steroid.

In another embodiment, a cream base of interest comprises a keratolytic agent.

In yet another specific embodiment, a cream base of interest comprises a depigmenting agent.

In another embodiment, a cream base of interest comprises two or more of a steroid, keratolytic agent and depigmenting agent.

An example of a steroid is fluocinolone, such as fluocinolone acetonide, of a keratolytic agent is tretinoin and of a depigmenting agent is hydroquinone.

In a more specific embodiment, the invention also provides a cream, which includes the inactive ingredients butylated hydroxytoluene, cetyl alcohol, citric acid, glycerin, glyceryl stearate, magnesium aluminum silicate, methyl gluceth-10, methylparaben, PEG-100 stearate, propylparaben, purified water, sodium metabisulfite, stearic acid and stearyl alcohol.

In a particular embodiment, the cream is a carrier that contains as an active ingredient, fluocinolone acetonide, hydroquinone, tretinoin and combinations thereof. For example, the cream can be Tri-Luma^{®} Cream, which is the first approved product to combine the standard depigmenting agent, hydroquinone, with tretinoin and a topical low-potency steroid that can be applied as a single preparation. The recommended course of therapy for Tri-Luma^{®} Cream is 8 weeks, and significant results have been seen after the first 4 weeks of treatment. Another advantage of the process of the invention is that by controlling the temperature at which the components, including hydroquinone, are added, the cream does not turn as brown, resulting in a more pleasing-colored product.

### DETAILED DESCRIPTION OF THE INVENTION

Creams are emulsions of hydrophilic and lipophilic (hydrophobic) components. Generally, an emulsifier or surface active agent is included to enhance the mixing of the reagents resulting in a stable emulsion.

The various compounds that comprise an inert carrier are generally known in the art. By "inert" is meant not having a pharmacologic activity. Typical examples of inert compounds comprising a cream base include cetyl alcohol, lanolin, glycerin, ethanol, EDTA, methyl paraben, zinc oxide, titanium dioxide, benzoic acid, carboxymethylcellulose, dimethylsulfoxide, polyethyleneglycol, petroleum, citric acid and stearic acid.

The instant invention relates to a method of making a cream base as a vehicle for one or more pharmacologically active agents for dermatologic applications. The method of interest comprises a particular order of adding and mixing of the ingredients of a cream. The hydrophilic ingredients, including water, are mixed. Heating may be used to facilitate dissolving and solubility to produce a solution. The lipophilic or hydrophobic ingredients are mixed separately. Heating may be used to facilitate mixing and homogenization.

The hydrophilic solution and the lipophilic solution then are mixed and blended. One or more pharmaceutically active agents then are added to the blended mixture. Then, one or more emulsifiers are added and the entire mixture blended to produce a dermatologic cream of interest.

The temperatures for heating the hydrophobic and hydrophilic solutions is that sufficient to facilitate the obtention of a homogeneous solution. Generally, a lower elevated temperature with longer mixing time is preferred. The temperature also may be limited by the properties of any one of the individual ingredients therein. Generally, the temperature does not exceed about 100°C. Preferably, the temperature does not exceed about 90°C or about 80°C or about 70°C or about 60°C. Generally, the temperature of heating need not be exact, at least within the accuracy of standard temperature measurement means.

The hydrophobic and hydrophilic solutions need not be heated to the same temperature. Having the same temperature facilitates the mixing of the two solutions. If the solutions are at different temperatures, the warmer solution is cooled to the temperature of the cooler solution prior to mixing.

The blended mixture optionally may be cooled prior to mixing in the one or more pharmacologically active agent or agents. The physical properties of the active agents may dictate a need for cooling.

Following that step and blending, one or more emulsifiers are added. The mixture is blended thoroughly to produce a cream of interest. If elevated, the temperature can be reduced during the blending.

As to the lipophilic ingredients, as known in the art, oils may be derived from animals, plants, nuts, petroleum etc. Those derived from animals, plant seeds and nuts are similar to fats and consequently, may contain a significant number of polar acid and/or ester groups. Alternatively, oils derived from petroleum are usually aliphatic or aromatic hydrocarbons that are essentially free of polar substitution.

Oil-based products which can be used include hydrocarbons or mineral fats obtained by the distillation of petroleum (petroleum jelly); vegetable oils and liquid triglycerides; animal fats or solid, natural triglycerides; and waxes or solid ethers of fatty acids, such as stearic acid and palmitic acid, and organic alcohols. Lanolin or wool fats made of fatty acids and cholesterol esters; and cetyl and stearyl alcohols, which are solid alcohols obtained by hydrogenation of their respective acids are also useable. Amphoteric compounds such as soaps or salts of fatty acids that may be acidic or basic depending on whether the lipophilic group is anionic or cationic, sulfated alcohols which are semi-synthetic substances and synthetic surface active agents are known in the art and also can be used. Glycerin is obtained from fats and, due to the hydrophobicity thereof, has the property of extracting water from the surface of mucosa or denuded skin. Glycerin does not damage intact skin because of having hydrophilic properties, and is a useful humectant.

Other materials that may be used in a topical preparation of interest include liquid alcohols, liquid glycols, liquid polyalkylene glycols, liquid esters, liquid amides, liquid protein hydrosylates, liquid alkylated protein hydrosylates, liquid lanolin and lanolin derivatives and other like materials. Particular examples include monohydric and polyhydric alcohols, e.g., ethanol, isopropanol, glycerol, sorbitol, 2-methoxyethanol, diethylene glycol, ethylene glycol, hexylene glycol, mannitol, cetyl alcohol and propylene glycol; ethers such as diethyl or dipropyl ether; polyethylene glycols and methoxypolyoxyethylenes; carbowaxes having molecular weights ranging from 200 to 20,000; polyoxyethylene glycerols; polyoxyethylene; sorbitols; and stearoyl diacetin.

The topical carriers often include both an alcohol and water so as to accommodate lipophilic and hydrophilic components. Other ingredients include buffers, such as sodium hydroxide, sodium citrate or tetrasodium EDTA; excipients; fragrances such as menthol; opacifiers such as zinc oxide, magnesium aluminum silicate and titanium dioxide; preservatives such as dichlorobenzyl alcohol, benzoic acid, methylparaben and phenyl carbinol; antioxidants; gelling agents such as petrolatum and mineral wax; thickening agents such as carboxymethylcellulose; stabilizers; surfactants; emollients; coloring agents and the like.

In addition, the topical carrier may include a penetration enhancer defined as a material that increases the permeability of the skin to one or more active agents so as to allow for cutaneous delivery of a pharmacologically active agent. Various compounds for enhancing the permeability of skin are known in the art. For example, dimethylsulfoxide (DMSO), dimethyl formamide (DMF) and N,N-dimethylacetamide (DMA), decylmethylsulfoxide, polyethylene glycol monolaurate and the 1-substituted azacycloheptan-2-ones.

A number of different emulsifiers or surfactants can be used to prepare a topical preparation of interest. Nonlimiting examples of amphoteric surfactants useful in the compositions of the present invention are disclosed in McCutcheon's, "Detergents and Emulsifiers", North American edition (1986) and McCutcheon's, "Functional Materials", North American edition (1992); both of which are incorporated by reference herein in their entirety. Surfactants that can used are the betaines, sultaines and hydroxysultaines. Examples of betaines include the higher alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, cetyl dimethyl betaine, lauryl bis-(2-hydroxyethyl) carboxymethyl betaine, steryl bis-(2-hydroxypropyl) carboxymethyl betaine, oleyl dimethyl gamma-carboxypropyl betaine, lauryl bis-(2-hydroxypropyl)alpha carboxyethyl betaine, coco dimethyl sulfopropyl betaine, stearyl dimethyl sulfopropyl betaine, stearyl betaine, lauryl dimethyl sulfoethyl betaine, lauryl bis-(2-hydroxyethyl) sulfopropyl betaine, amidobetaines, amidosulfobetaines, oleyl betaine and cocamidopropyl betaine. Examples of sultaines and hydroxysultaines include cocamidopropyl hydroxysultaine. Examples of other amphoteric surfactants are alkyliminoacetates, iminodialkanoates and aminoalkanoates.

Examples of anionic surfactants also are disclosed in McCutcheon's, "Detergents and Emulsifiers", North American edition (1986) and McCutcheon's, "Functional Materials", North American edition (1992). Examples include the alkoyl isothionates, the alkyl and alkyl ether sulfates, such as, ammonium cocoyl isothionate, sodium cocoyl isothionate, sodium lauroyl isothionate, sodium stearoyl isothionate and mixtures thereof, the sarcosinates, such as sodium lauroyl sarcosinate, sodium cocoyl sarcosinate and ammonium lauroyl sarcosinate, sodium lauryl sulfate, ammonium lauryl sulfate, ammonium cetyl sulfate, sodium cetyl sulfate, sodium stearyl sulfate, ammonium cocoyl isethionate, sodium lauroyl isethionate, sodium lauroyl sarcosinate and mixtures thereof.

Other emulsifiers includes tricetareth-4-phosphate, sodium laureth-4-phosphate or oleth-3.

Examples of non-ionic emulsifiers include sorbitan monostearate, glyceryl monostearate, polysorbates, polyethylene derivatives of fatty alcohols, polyoxyethylene ethers of fatty alcohols, such as polyoxyethylene cetyl ether, polyoxyethylene oleyl ether, polyoxyethylene nonylphenyl ether and the like, sorbitan stearate, glyceryl stearate, C₁₂-C₁₈ fatty alcohols, esters and ethers thereof, aliphatic fatty alcohols such as cetyl alcohol or stearyl alcohol or a mixture of the two, fatty alcohols or α-diols oxyethylenated or polyglycerolated such as oleyl alcohol polyoxyethylenated with 10 moles of ethylene oxide, 1,2-octadecanediol polyglycerolated with 2 or 7 moles of glycidol, cyclic fatty alcohols, glycol esters of fatty acids such as ethylene glycol stearate, the monostearates or distearates of glycerol, the polyethylene glycol esters of fatty acids such as polyethylene glycol stearates, the fatty esters of sorbitan oxyethylenated or not and sold under the trade name of Tweens or Spans, the fatty esters of sucrose, the fatty esters of glucose derivatives such as methylglucoside sesquistearate and methylglucoside sesquistearate polyoxyethylenated with 20 moles of ethylene oxide, Arlacel 165 and Myrj 52, fatty alcohols having 10 to 20 carbon atoms, fatty alcohols having 10 to 20 carbon atoms condensed with 2 to 20 moles of ethylene oxide or propylene oxide, alkyl phenols with 6 to 12 carbon atoms in the alkyl chain condensed with 2 to 20 moles of ethylene oxide, mono-fatty acid and di-fatty acid esters of ethylene oxides, mono-fatty acid and di-fatty acid esters of ethylene glycol wherein the fatty acid moiety contains from 10 to 20 carbon atoms, diethylene glycol, polyethylene glycols of molecular weight 200 to 6000, propylene glycols of molecular weight 200 to 3000, glycerol, sorbitol, sorbitan, polyoxyethylene sorbitol, polyoxyethylene sorbitan and hydrophilic wax esters, polyoxyethylene fatty alcohol ethers, polyoxyethylene fatty acid ester, polyoxyethylene sorbitan fatty acid esters, sorbitan fatty acid esters, polyoxyethylene glycol fatty acid esters and polyol fatty acid esters.

Examples of cationic emulsifiers include quaternized ammonium bromide and chloride salts, cetyltrimethylammonium chloride, benzalkonium chloride and cetyl pyridinium chloride, aliphatic amines having fatty chains, e.g., oleylamine and dihydroabietylamine; quaternary ammonium compounds, e.g., lauryl dimethylbenzyl ammonium chloride, amides derived from amino alcohols, e.g., N-aminoethyl oleylamide, n-(stearoyl-colamino-formylmethyl) pyridinium chloride, N-soya-N-ethyl morpholinium ethosulphate, alkyl dimethyl benzyl ammonium chloride, di-isobutylphenoxyethoxyethyl dimethyl benzyl ammonium chloride, cetyl pyridinium chloride, N-(stearoyl-colamino- formylmethyl) pyridinium chloride, N-soya-N-ethyl morpholinium ethosulfate, alkyl dimethyl benzyl ammonium chloride, (diisobutyl-phenoxy-ethoxy) ethyl dimethyl benzyl ammonium chloride, PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, ammonium halides, more especially chlorides and bromides, such as alkyl trimethylammonium chlorides, dialkyl dimethylammonium chlorides and trialkyl methylammonium chlorides, for example, stearyl trimethylammonium chloride, distearyl dimethylammonium chloride, lauryl dimethylammonium chloride, lauryl dimethyl benzylammonium chloride and tricetyl methylammonium chloride, quaternized protein hydrolyzates or protein hydrolyzates derivatized with amino groups which are marketed, for example, under the names Lamequat^{®} and Mackpro^{®}, stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, and stearamidopropyl dimethyl ammonium lactate.

The compositions of the instant invention can comprise a wide range of additional components. The "CTFA Cosmetic Ingredient Handbook", Second edition, 1992, which is incorporated by reference herein in its entirety, describes a wide variety of cosmetic and pharmaceutical ingredients commonly used in the skin care industry, which are suitable for use in the compositions of the instant invention. Examples of functional classes of ingredients are absorbents, abrasives, anti-acne agents, anticaking agents, antifoaming agents, antimicrobial agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, platicizers, preservatives, propellants, reducing agents, skin bleaching agents, skin conditioning agents (emollients and humectants), skin protectants, solvents, foam boosters, hydrotropes, solubilizing agents, suspending agents (nonsurfactant), sunscreen agents, ultraviolet light absorbers and viscosity increasing agents (aqueous and nonaqueous).

The various starting materials for making a topical preparation are known in the art and reference can be made to known treatises, as well as U.S. Patent Nos. 6,013,271; 6,267,985; 4,992,478; 5,645,854; 5,811,111; and 5,851,543.

A number of pharmaceutically active agents can be used in the dermatologic preparations of interest. Thus, any of the known antibiotics, anti-acne agents, antineoplastic agents, bleaching agents, keratolytic agents, anti-inflammatories, antifungal analgesics and so on, can be used.

A wide variety of cytostatic agents may be used. Examples include cytostatic agents, alkylating agents, enzyme inhibitors, proliferation inhibitors, DNA synthesis inhibitors, lytic agents, DNA intercalators, antimetabolites and the like. Illustrative agents include steroids, paclitaxel, ionomycin, etoposide, nitrosoureas such as carmustine (BCNU), doxorubicin, daunoxubicin, actinomycin D, meclorethamine, busulfan, CCNU, Me-CCNU, chlorambucil, cactinomycin, carzinophilin, chlornaphazine, 6-chloropurine, azathioprine, fluorouracil, hydroxyurea, thioquanine, campothecin, mitomycin, lomustine (CCNU), semustine (Me-CCNU), cantharidin, camptothecin, carboplatin, ricin, pseudomonas exotoxin, interferons, interleukins, tumor necrosis factors, vincristine, mitotane melphalan, methchlorethamine, plicamycin, nitracine, nitoxantrone, methotrexate, nogalamycin, streptonigrin, streptozocin, tegafur, tetramin, testolactone demecolcine and dactinomycin. Other compounds that can be used include cyctophamide, cyclosporin, amsacrine, biantrene hydrochloride, camostat mesylate, campothecin, enocitabine, etoposide, epirubicin hydrochloride, fludarabine phosophate, flutamide, fotemustine, idarubicin hydrochloride, ionomycin, onidamine, mitoxantrone hydrochloride, nilutamide, paclitaxel, pirarubicin, toremifene, vinorelbine, didemnin, bactracyclin, mitoquidone, penclomedine, phenazinomycin, U-73975, saintopin, 9-aminocamptothecin, amonafide, merbarone and the like. Additional agents that can be used include mitomycin C, cisplatin, mechlorethamine, daunorubicin, carmustine, pyrazine diazohydroxide, fumagillin analog FF- 11 1142, rhyzoxin, dynemicin A, chlorambucil, semustine and the like.

Suitable keratolytics include salicylic acid, derivatives of salicylic acid such as 5-octanoyl salicylic acid, and resorcinol; retinoids such as retinoic acid and derivatives thereof (e.g., cis and trans); sulfur-containing D and L amino acids and derivatives and salts thereof, particularly N-acetyl derivatives, such as N-acetyl-L-cysteine; lipoic acid; antibiotics and antimicrobials such as benzoyl peroxide, actopirox, tetracycline, trichlorobanilide, azelaic acid, phenoxyethanol, phenoxypropanol, phenoxyisopropanol, ethyl acetate, clindamycin and meclocycline; sebostats such as flavonoids; and bile salts such as scymnol sulfate and derivatives thereof, deoxycholate and cholate.

Examples of antiwrinkle and anti-skin atrophy actives that can be used in the topical preparations of interest include retinoic acid and derivatives; retinol; retinyl esters; salicylic acid and derivatives thereof; sulfur-containing D and I, amino acids and their derivatives and salts, particularly the N-acetyl derivatives, thiols, e.g. ethane thiol; alpha-hydroxy acids, e.g. glycolic acid, and lactic acid; phytic acid, lipoic acid; lysophosphatidic acid, and skin peel agents, e.g., phenol.

Examples of non-steroidal anti-inflammatories that can be used in the instant invention include propionic acid derivatives; acetic acid derivatives; fenamic acid derivatives; biphenylcarboxylic acid derivatives; and oxicams, and include acetyl salicylic acid, ibuprofen, naproxen, benoxaprofen, fluhiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen and bucloxis acid.

Examples of topical anesthetic drugs that can be used in the topical preparation of interest include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof.

Corticosteroids including halogenated corticosteroids that can be used in the topical preparations of interest generally are known and are commercially available. Examples include cortisone, hydrocortisone and derivatives thereof including cortodoxone, flucetonide, fludrocortisone acetate, flurandrenolone acetonide, medrysone; prednisone, prednisolone and derivatives thereof including amcinafal, amcinafide, betamethasone benzoate, valerate and dipropionate, chloroprednisone acetate, descinalone acetonide, desonide, dexamethasone, dichlorisone acetate, difluprednate, flucloronide, flumethasone, flunisolide acetate, fluocinolone acetonide, fluocinonide, fluocortolone, fluorometholone, fluperoline acetate, fluprednisolone valerate, meprednisone, methyl prednisolone, paramethasone acetate, prednisolomate, prednisolone acetate, butylacetate and phosphate sodium, triamcinolone acetonide, hexacetonide, diacetate, hydrocortisone butyrate, flumethasone pivalate, halcininide and clobetasol propionate.

Examples of other active ingredients that can be used in a topical preparation of interest include acebutolol, acetaminophen, acetohydoxamic acid, acetophenazine, acyclovir, allopurinol. alprazolam, aluminum hydroxide, amantadine, ambenonium, amiloride, aminobenzoate potassium, amobarbital, amoxicilin, amphetamine, ampicillin, androgens, anesthetics, anticoagulants, anticonvulsants, antithyroids, appetite suppressants, aspirin, atenolol, atropine, azatadine, bacampicillin, baclofen, beclornethasone, belladonna, bendroflumethiazide, benzoyl peroxide, benzthiazide, benztropine, bethanechol, biperiden, bisacodyl, bromocriptine, bromodiphenhydramine, brompheniramine, buclizine, bumetanide, busulfan, butabarbital, butaperazine, caffeine, calcium carbonate, captopril, arbamazepine, carbenicillin, carbidopa, levodopa, carbinoxamine inhibitors, carbonic anhydrase, carisoprodol, carphenazine, cascara, cefaclor, cefadroxil, cephalexin, cephradine, chlophedianol, chloral hydrate, chlorambucil, chloramphenicol, chlordiazepoxide, chloroquine, chlorothiazide, chlorotianisene, chlorpheniramine, chlorpromazine, chlorpropamide, chlorprothixene, chlorthalidone, chlorzoxazone, cholestyramine, cimetidine, cinoxacin, clemastine, clidinium, clindamycin, clofibrate, clomiphere, clonidine, clorazepate, cloxacillin, colchicine, coloestipol, estrogens, contraceptives, androgens, cromolyn, cyclacillin, cyclandelate, cyclizine, cyclobenzaprine, cyclophosphamide, cyclothiazide, cycrimine, cyproheptadine, danazol, danthron, dantrolene, dapsone, dextroamphetamine, dexchlorpheniramine, dextromethorphan, diazepan, dicloxacillin, dicyclomine, diethylstilbestrol, diflunisal, digitalis, diltiazen, dimenhydrinate, dimethindene, diphenhydramine, diphenidol, diphenoxylate, diphenylopyraline, dipyradamole, disopyramide, disulfiram, divalporex, docusate calcium, docusate potassium, docusate sodium. doxyloamine, dronabinol ephedrine, epinephrine, ergoloidmesylates, ergonovine, ergotamine, erythromycins, estropipute, etharynic acid, ethchlorvynol, ethopropazine, ethosaximide, ethotoin, fenoprofen, ferrous fumarate, ferrous gluconate, ferrous sulfate, flavoxate, flecainide, fluphenazine, fluprednisolone, flurazepam, folic acid, furosemide, gemfibrozil, glipizide, glyburide, glycopyrrolate, gold compounds, griseofuwin, guaifenesin, guanabenz, guanadrel, guanethidine, halazepam, haloperidol, hetacillin, hexobarbital, hydralazine, podofilox (Oclassen Dermatologics), podophyllin (Paddock Labs), imiquimod (3M Pharmaceuticals), hydrochlorothiazide, hydroflunethiazide, hydroxychloroquine, hydroxyzine, hyoscyamine, ibuprofen, indapamide, indomethacin, insulin, iofoquinol, iron-polysaccharide, isoetharine, isoniazid, isopropamide, isoproterenol, isotretinoin, isoxsuprine, kaolin, pectin, ketoconazole, lactulose, levodopa, lincomycin, liothyronine, liotrix, lithium, loperamide, lorazepam, magnesium hydroxide, magnesium sulfate, magnesium trisilicate, maprotiline, meclizine, medofenamate, medroxyproyesterone, melenamic acid, melphalan, mephenytoin, mephobarbital, meprobamate, mercaptopurine, mesoridazine, metaproterenol, metaxalone, methamphetamine, methaqualone, metharbital, methenamine, methicillin, methocarbamol, methotrexate, methsuximide, methyclothinzide, methylcellulose, methyldopa, methylergonovine, methylphenidate, methylprednisolone, methysergide, metoclopramide, metolazone, metoprolol, metronidazole, minoxidil, mitotane, monamine oxidase inhibitors, nadolol, nafcillin, nalidixic acid, naproxen, narcotic analgesics, neomycin, neostigmine, niacin, nicotine, nifedipine, nitrates, nitrofurantoin, nomifensine, nylidrin, nystatin, orphenadrine, oxacillin, oxazepam, oxprenolol, oxymetazoline, oxyphenbutazone, pancrelipase, pantothenic acid, papaverine, paraaminosalicylic acid, paregoric, pemoline, penicillamine, penicillins, pentobarbital, perphenazine, phenacetin, phenazopyridine, pheniramine, phenobarbital, phenolphthalein, phenprocoumon, phensuximide, phenylbutazone, phenylephrine, phenylpropanolamine, phenyl toloxamine, phenytoin, pilocarpine, pindolol, piperacetatine, piroxicam, poloxamer, polycarbophil calcium, polythiazide, potassium supplements, pruzepam, prazosin, primidone, probenecid, probucol, procainamide, procarbazine, prochlorperazine, procyclidine, prornazine, promethazine, propantheline, propranolol, pseudoephedrine, psoralens, psyllium, pyridostigmine, pyrodoxine, pyrilamine, pyrvinium, quinestrol, quinethazone, quinidine, quinine, ranitidine, rauwolfia alkaloids, riboflavin, rifampin, ritodrine, salicylates, scopolamine, secobarbital, senna, sannosides, simethicone, sodium bicarbonate, sodium phosphate, sodium fluoride, spironolactone, sucrulfate, sulfacytine, sulfamethoxazole, sulfasalazine, sulfinpyrazone, sulfisoxazole, sulindac, talbutal, tamazepam, terbutaline, terfenadine, terphinhydrate, teracyclines, thiabendazole, thiamine, thioridazine, thiothixene, thyroglobulin, thyroid, thyroxine, ticarcillin, timolol, tocainide, tolazamide, tolbutamide, tolmetin, trozodone, tretinoin, triamcinolone, trianterene, triazolam, trichlormethiazide, tricyclic antidepressants, trifluoperazine, triflupromazine, trihexyphenidyl, trimeprazine, trimethobenzamine, trimethoprim, tripclennamine, triprolidine, valproic acid, verapamil and xanthine.

The amounts of the inert ingredients and active agent(s) in the dermatologic preparation of interest generally are known in the art. It is within the ambit of the artisan to derive particular amounts of the ingredients to obtain a cream of interest.

The particular amount of any one ingredient used is not substantially critical and the amounts used are at the accuracy of the measuring or dispensing means known in the art.

In one embodiment of the invention, approximately 344.8 kg of water, 15.0 kg magnesium aluminum silicate, and 0.2 kg butylated hydroxytoluene are first combined and mixed at 75-80°C to form the aqueous phase. The mixing can be by side scrape agitation at a fixed speed. The resulting aqueous phase is a suspension.

Second, approximately 20.0 kg of cetyl alcohol, 15.0 kg of stearic acid, 20.0 kg of stearyl alcohol, 25.0 kg of methyl gluceth-10, 0.9 kg of methylparaben, 0.1 kg of propylparaben, and 20.0 kg of glycerin are mixed together at medium speed at about 75-80°C to form the non-aqueous phase. The mixing can be at medium speed in a Lightnin^{®} mixer. The resulting non-aqueous phase is a suspension. The second step can be performed before, after or concurrently with the first step.

Then, the non-aqueous phase is added to the aqueous phase and the combined biphasic mixture is cooled to a temperature in the range of 68°C to 72°C, or about 70°C, after which about 17.5 kg of Arlacel^{®} 165, 0.25 kg tretinoin and 0.050 kg fluocinolone acetonide are added and stirred with cooling. When the mixture reaches 60°C, 0.25 kg citric acid is added with mixing and cooling. When the temperature reaches 55°C, 20.0 kg hydroquinone is added with mixing and cooling. When the temperature reaches about 50°C, the mixture is homogenized with a homogenizer, with continued cooling. When the mixture reaches 45°C, 1.0 kg of sodium metabisulfite is added with stirring and cooling. Typically, the sodium metabisulfite is added about 30 minutes after the addition of the hydroquinone. The mixing can be at fixed speed in a side scrape agitator. The resulting composition of matter is an emulsion, i.e., a cream.

The presence of sodium metabisulfite in the cream prevents the oxidation of hydroquinone. The addition of sodium metabisulfite as the cream is cooling advantageously results in a well-mixed composition of matter, with the sodium metabisulfite evenly mixed throughout the cream and preventing the oxidation of the hydroquinone throughout the cream. Another advantage of the process of the invention is that by controlling the temperature at which the components, including hydroquinone, are added, the cream does not turn as brown, resulting in a more pleasing-colored product.

The addition of the emulsifier following the mixing of the non-aqueous and aqueous phases is advantageous for the making of the pharmaceutical composition of the invention. When a standard technique of adding the emulsifier to the non-aqueous phase and then mixing with the aqueous phase was used, no emulsion formed. However, when the emulsifier was added to the mixture of the non-aqueous and aqueous phases with cooling, according to the method of the invention, a useful emulsion did form. This emulsion formed even though the relative proportion of the non-aqueous and aqueous phases according to the successful method of the invention was the same as when an emulsion did not form using the standard technique of adding a non-aqueous phase containing an emulsifier to an aqueous phase.

The resulting TRI-LUMA^{®} Cream contains fluocinolone acetonide, hydroquinone and tretinoin in a hydrophilic cream base for topical application. Each gram of TRI-LUMA^{®} Cream contains as active ingredients, fluocinolone acetonide 0.01% (0.1 mg), hydroquinone 4% (40 mg), and tretinoin 0.05% (0.5 mg), and as inactive ingredients, butylated hydroxytoluene, cetyl alcohol, citric acid, glycerin, glyceryl stearate, magnesium aluminum silicate, methyl gluceth-10, methylparaben, PEG-100 stearate, propylparaben, purified water, sodium metabisulfite, stearic acid, and stearyl alcohol, see TABLE 1.

**TABLE 1**

| **Ingredient** | **500 kg Batch Quantity** | **800 kg Batch Quantity** | **Formula** |
|---|---|---|---|
| magnesium aluminum silicate NF | 15 kg | 24 kg | 3.00% |
| butylated hydroxytoluene NF | 200 g | 320 g | 0.04% |
| cetyl alcohol NF | 20 kg | 32 kg | 4.00% |
| stearic acid NF | 15 kg | 24 kg | 3.00% |
| stearyl alcohol NF | 20 kg | 32 kg | 4.00% |
| methylparaben NF | 900 g | 1,440 g | 0.18% |
| propylparaben NF | 100 g | 160 g | 0.02% |
| Arlacel® 165 [glycerol stearate and PEG-100 stearate glycerol monostearate] | 17.5 kg | 28 kg | 3.50% |
| methyl gluceth-10 | 25 kg | 40 kg | 5.00% |
| glycerin USP | 20 kg | 32 kg | 4.00% |
| tretinoin USP | 250 g | 400 g | 0.05% |
| fluocinolone acetonide USP | 50 g | 80 g | 0.01% |
| citric acid USP | 250 g | 400 g | 0.05% |
| hydroquinone USP | 20 kg | 32 kg | 4.00% |
| sodium metabisulfite NF | 1 kg | 1.6 kg | 0.20% |
| purified water USP | 344.8 kg | 551.6 kg | 68.95% |
| **TOTAL** | | | **100.00%** |

Fluocinolone acetonide is a synthetic fluorinated corticosteroid for topical dermatological use and is classified therapeutically as an anti-inflammatory. It is a white crystalline powder that is odorless and stable in light. The chemical name for fluocinolone acetonide is (6,11,16)-6,9-difluoro-11,21-dihydroxy-16,17-[(1-methylethylidene)bis(oxy)]-pregna-1,-4-diene-3,20-dione. The molecular formula is C₂₄H₃₀F₂O₆ and molecular weight is 452.50.

Hydroquinone is classified therapeutically as a depigmenting agent. It is prepared from the reduction of *p*-benzoquinone with sodium bisulfite. It occurs as fine white needles that darken on exposure to air. The chemical name for hydroquinone is 1,4-benzenediol. The molecular formula is C₆H₆O₂ and molecular weight is 110.11.

Tretinoin is all-*trans*-retinoic acid formed from the oxidation of the aldehyde group of retinene to a carboxyl group. It is highly reactive to light and moisture. Tretinoin is classified therapeutically as a keratolytic. The chemical name for tretinoin is:
(*all*-*E*)-3,7-dimethyl-9-(2,6,6-trimethyl-1-cyclohexen-1-yl)-2,4,6,8-nonatetraenoic acid. The molecular formula is C₂₀H₂₈O₂ and molecular weight is 300.44.

TRI-LUMA™ Cream is typically supplied in 30 g aluminum tubes, NDC 0299-5950-30, and is stored at controlled room temperature 68 to 77°F (20-25°C).

The details of one or more embodiments of the invention are set forth in the accompanying description above. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs. All patents and publications cited in this specification are incorporated by reference.

The following EXAMPLES are presented to more fully illustrate the preferred embodiments of the invention. These EXAMPLES should in no way be construed as limiting the scope of the invention, as defined by the appended claims.

### EXAMPLE I

### HUMAN PHARMACOKINETICS

Percutaneous absorption of unchanged tretinoin, hydroquinone and fluocinolone acetonide into the systemic circulation of two groups of healthy volunteers (Total n=59) was found to be minimal following 8 weeks of daily application of 1 g (Group I, n=45) or 6 g (Group II, n=14) of TRI-LUMA^{®} Cream.

For tretinoin quantifiable plasma concentrations were obtained in 57.78% (26 out of 45) of Group I and 57.14% (8 out of 14) of Group II subjects. The exposure to tretinoin as reflected by the Cₘₐₓ values ranged from 2.01 to 5.34 ng/mL (Group I) and 2.0 to 4.99 ng/mL (Group II). Thus, daily application of TRI-LUMA^{®} Cream resulted in a minimal increase of normal endogenous levels of tretinoin. The circulating tretinoin levels represent only a portion of total tretinoin-associated retinoids, which would include metabolites of tretinoin and that sequestered into peripheral tissues.

For hydroquinone quantifiable plasma concentrations were obtained in 18% (8 out of 44) Group I subjects. The exposure to hydroquinone as reflected by the Cₘₐₓ values ranged from 25.55 to 86.52 ng/mL. All Group II subjects (6g dose) had undetectably low post-dose plasma concentrations.

For fluocinolone acetonide, Groups I and II subjects had undetectably low post-dose plasma concentrations.

The following tests may be helpful in evaluating patients: (a) ACTH or cosyntropin stimulation tests; (b) the A.M. plasma cortisol test; and (c) the urinary free cortisol test.

### EXAMPLE II

### HUMAN CLINICAL STUDIES

Two efficacy and safety studies were conducted in 641 melasma patients between the ages of 21 to 75 years, having skin phototypes I-IV and moderate to severe melasma of the face. TRI-LUMA^{®} Cream was compared with three possible combinations of two of the three active ingredients [(1) hydroquinone 4% (HQ) + tretinoin 0.05% (RA); (2) fluocinolone acetonide 0.01% (FA) + tretinoin 0.05% (RA); (3) fluocinolone acetonide 0.01% (FA) + hydroquinone 4% (HQ)], contained in the same vehicle as TRI-LUMA^{®} Cream.

The patients were instructed to apply their study medication each night, after washing their face with a mild soapless cleanser, for 8 weeks. The patients were also instructed to apply a thin layer of study medication to the hyperpigmented lesion, making sure to cover the entire lesion including the outside borders extending to the normal pigmented skin. The patients were provided a mild moisturizer for use as needed and a sunscreen with SPF 30 for daily use. Moreover, the patients were instructed to avoid sunlight exposure to the face, wear protective clothing Protective clothing and avoidance of sunlight exposure to the face was recommended.

The patients were evaluated for melasma severity at baseline and at weeks 1, 2, 4, and 8 of treatment. Primary efficacy was based on the proportion of patients who had an investigators' assessment of treatment success, defined as the clearing of melasma at the end of the eight-week treatment period. The majority of patients enrolled in the two studies were white (approximately 66%) and female (approximately 98%). TRI-LUMA^{®} Cream was demonstrated to be significantly more effective than any of the other combinations of the active ingredients.

Patients experienced improvement of their melasma with the use of TRI-LUMA^{®} Cream as early as 4 weeks. However, among 7 patients who had clearing at the end of 4 weeks of treatment with TRI-LUMA^{®} Cream, 4 of them did not maintain the remission after an additional 4 weeks of treatment.

After 8 weeks of treatment with the study drug, patients entered into an open-label extension period in which TRI-LUMA^{®} Cream was given on an as-needed basis for the treatment of melasma. In studies, after 8 weeks of treatment with TRI-LUMA^{®} Cream, most patients had at least some improvement. Some had their dark spots clear up completely (38% in one study and 13% in another). In most patients treated with TRI-LUMA^{®} Cream, their melasma came back after treatment. The remission periods appeared to shorten between progressive courses of treatment. Additionally, few patients maintained complete clearing of melasma (approximately 1 to 2%).

**TABLE 2**

| **Investigators' Assessment of Treatment Success* At the End of 8 Weeks of Treatment** | | | | | |
|---|---|---|---|---|---|
| | | **TRI**-**LUMA^{®} Cream** | **HQ**+**RA** | **FA+RA** | **FA+HQ** |
| Study No. 1 | Number of Patients | 85 | 83 | 85 | 85 |
| | Number of Successes | 32 | 12 | 0 | 3 |
| | Proportion of Successes | 38% | 15% | 0% | 4% |
| | P-value | | <0.001 | <0.001 | 0.001 |
| Study No. 2 | Number of Patients | 76 | 75 | 76 | 78 |
| | Number of Successes | 10 | 3 | 3 | 1 |
| | Proportion of Successes | 13% | 4% | 4% | 1% |
| | P-value# | | 0.045 | 0.042 | 0.005 |

| | | | | | |
|---|---|---|---|---|---|
| *Treatment success was defined as melasma severity score of zero (melasma lesions cleared of hyperpigmentation). #P-value is from Cochran-Mantel-Haenszel chi-square statistics controlling for pooled investigator and comparing TRI-LUMA^{®} Cream to the other treatment groups. | | | | | |

Based on melasma severity at the beginning of the trial, 161 patients were assessed for improvement at day 56 of treatment. 61% (99 patients) experienced symptom improvement from "moderate" to "mild" or "cleared," and 68% (25) showed improvement from "severe" to "mild" or "cleared" over the 8-week treatment period as shown in TABLE 3.

**TABLE 3**

| **Investigators' Assessment of Change in Melasma Severity from Baseline to Day 56 of Treatment (combined results from studies 1 and 2)** | | | | | | | |
|---|---|---|---|---|---|---|---|
| | | | **Number(%) of Patients at Day 56^{a}** | | | | |
| | **Baseline** | | **Cleared^{b} N(%)** | **Mild^{b} N(%**) | **Moderate^{b} N(%)** | **Severe^{d} N(%)** | **Missin N(%)** |
| | **Severity Rating** | **N** | | | | | |
| Tri-Luma^{®} | Moderate | 124 | 36 (29) | 63 (51) | 18(16) | 0(0) | 7 (6%) |
| Cream N =161 | Severe | 37 | 6(16) | 19(51) | 9 (24) | 2 (5) | 1 (3%) |

| | | | | | | | |
|---|---|---|---|---|---|---|---|
| ^{a} Assessment based on patients with severity scores at day 56. Percentages are based on the total number in the treatment group population. ^{b} Does not include patients who cleared before day 56 or were missing from the day 56 assessment. Assessment scale: Cleared (melasma lesions approximately equivalent to surrounding normal skin or with minimal residual hyperpigmentation); Mild (slightly darker than the surrounding normal skin); Moderate (moderately darker than the surrounding normal skin); Severe (markedly darker than the surrounding normal skin). | | | | | | | |

### EXAMPLE III

### ADVERSE REACTIONS IN HUMANS

In a patch test study to determine sensitization potential in 221 healthy volunteers, three volunteers developed sensitivity reactions to TRI-LUMA^{®} Cream or its components.

In the controlled clinical trials, adverse events were monitored in the 161 patients who used TRI-LUMA^{®} Cream once daily during an 8-week treatment period. There were 102 (63%) patients who experienced at least one treatment-related adverse event during these studies. The most frequently reported events were erythema, desquamation, burning, dryness, and pruritus at the site of application. The majority of these events were mild to moderate in severity. Adverse events reported by at least 1% of patients and judged by the investigators to be reasonably related to treatment with TRI-LUMA^{®} Cream from the controlled clinical studies are summarized (in decreasing order of frequency) as follows:

**TABLE 4**

| **Incidence and Frequency of Treatment-Related Adverse Events with TRI-LUMA^{®} Cream In At Least 1% or More of Patients (N=161)** | | |
|---|---|---|
| **Adverse Event** | **Number** | **(%) of Patients** |
| Erythema | 66 | (41%) |
| Desquamation | 61 | (38%) |
| Burning | 29 | (18%) |
| Dryness | 23 | (14%) |
| Pruritus | 18 | (11%) |
| Acne | 8 | (5%) |
| Paresthesia | 5 | (3%) |
| Telangiectasia | 5 | (3%) |
| Hyperesthesia | 3 | (2%) |
| Pigmentary changes | 3 | (2%) |
| Irritation | 3 | (2%) |
| Papules | 2 | (1%) |
| Acne-like rash | 1 | (1%) |
| Rosacea | 1 | (1%) |
| Dry mouth | 1 | (1%) |
| Rash | 1 | (1%) |
| Vesicles | 1 | (1%) |

In an open-label long-term safety study, patients who have had cumulative treatment of melasma with TRI-LUMA^{®} Cream for 6 months showed a similar pattern of adverse events as in the 8-week studies. The following local adverse reactions have been reported infrequently with topical corticosteroids. They may occur more frequently with the use of occlusive dressings, especially with higher potency corticosteroids. These reactions are listed in an approximate decreasing order of occurrence: burning, itching, irritation, dryness, folliculitis, acneiform eruptions, hypopigmentation, perioral dermatitis, allergic contact dermatitis, secondary infection, skin atrophy, striae, and miliaria.

All references cited herein are herein incorporated by reference in entirety.

The foregoing description has been presented only for the purposes of illustration and is not intended to limit the invention to the precise form disclosed, but by the claims appended hereto.

The following paragraphs describe embodiments of the invention.
1. A process of making a pharmaceutical composition for topical application, the composition being an emulsion comprising water and at least one active ingredient comprising:
   (a) combining water and at least one hydrophilic compound to form an aqueous phase;
   (b) combining at least two hydrophobic compounds to form a non-aqueous phase;
   (c) combining the aqueous phase and non-aqueous phase to form a biphasic mixture;
   (d) adding at least one active ingredient;
   (e) adding at least one emulsifier; and
   (f) homogenizing the mixture to form the emulsion.
2. The process of paragraph 1, wherein the aqueous phase consists essentially of water, magnesium aluminum silicate, glycerin and butylated hydroxytoluene.
3. The process of paragraph 1, wherein the non-aqueous phase comprises at least two hydrophobic compounds selected from the group consisting of cetyl alcohol, stearic acid, stearyl alcohol, methylparaben and propylparaben.
4. The process of paragraph 1, wherein the non-aqueous phase consists essentially of cetyl alcohol, stearic acid, stearyl alcohol, methylparaben and propylparaben.
5. The process of paragraph 1, wherein said combining step (a), (b) or both are conducted at a temperature greater than room temperature to yield a heated phase.
6. The process of paragraph 5, wherein said heated phase is cooled prior to said combining step (c).
7. The process of paragraph 5, wherein said heated biphasic mixture is cooled prior to step (d).
8. The process of paragraph 1, wherein said at least one active ingredient comprises a steroid, a keratolytic agent, a depigmenting agent or combination thereof.
9. The process of paragraph 8, wherein said steroid is betamethasone, fluocinolone, cortisone, hydrocortisone, prednisone, prednisolone, dexamethasone or clobetasol.
10. The process of paragraph 8, wherein said keratolytic agent is podofilox, podophyllin, imiquimod, an interferon or a retinoic acid derivative.
11. The process of paragraph 8, wherein said depigmenting agent is monobenzone or hydroquinone.
12. The process of paragraph 1, wherein the emulsifier is glyceryl stearate, polyethylene glycol (PEG) stearate or a combination thereof.
13. The process of paragraph 12, wherein the glyceryl stearate, polyethylene glycol stearate or combination thereof is Arlacel^{®} 165.
14. A dermatologic preparation made by the process of any one of paragraphs 1-13.
15. A pharmaceutical composition for topical application, the composition being an emulsion comprising at least one active ingredient selected from the group consisting of fluocinolone acetonide, hydroquinone and tretinoin, the composition made by a process comprising the steps of:
   (a) combining water and at least one hydrophilic compound while heating at a temperature no greater than about 80°C to form an aqueous phase;
   (b) combining at least two hydrophobic compounds while heating at a temperature no greater than about 80°C to form a non-aqueous phase;
   (c) combining the aqueous phase and non-aqueous phase to form a biphasic mixture;
   (d) stirring and cooling the biphasic mixture to a temperature about 70°C, then adding said at least one active ingredient;
   (e) while stirring and cooling the mixture to a temperature about 70°C, adding at least one emulsifier; and
   (f) homogenizing the mixture to form the emulsion.
16. The pharmaceutical composition of paragraph 15 , wherein the active ingredient is selected from the group consisting of about 0.01% by weight fluocinolone acetonide, about 4% by weight hydroquinone and about 0.05% by weight tretinoin.
17. The pharmaceutical composition of paragraph 15 , wherein the active ingredient is selected from the group consisting of about 4% by weight hydroquinone and about 0.05% by weight tretinoin; about 0.01% by weight fluocinolone acetonide and about 0.05% by weight tretinoin; and about 0.01% by weight fluocinolone acetonide and about 4% by weight hydroquinone.
18. The pharmaceutical composition of paragraph 15, wherein the active ingredient comprises about 0.01% by weight fluocinolone acetonide, about 4% by weight hydroquinone and about 0.05% by weight tretinoin.
19. The pharmaceutical composition of paragraph 15, wherein the emulsifier is glyceryl stearate, polyethylene glycol (PEG) stearate or a combination thereof.
20. The pharmaceutical composition of paragraph 19, wherein the glyceryl stearate, polyethylene glycol stearate or combination thereof is Arlacel^{®} 165.
21. The pharmaceutical composition of paragraph 15, wherein when hydroquinone is at least one active agent, said hydroquinone is added following step (e).
22. The pharmaceutical composition of paragraph 21, further comprising following step (e) while stirring adding sodium metabisulfite to said emulsion.
23. A pharmaceutical composition, consisting essentially of about 0.01 weight % fluocinolone acetonide; about 4 weight % hydroquinone; about 0.05 weight % tretinoin; about 0.04 weight % butylated hydroxytoluene; about 4 weight % cetyl alcohol; about 0.05 weight % citric acid; about 4 weight % glycerin; about 3 weight % magnesium aluminum silicate; about 5 weight % methyl gluceth; about 0.18 weight % methylparaben; about 3.5 weight % glyceryl stearate, polyethylene glycol (PEG) stearate or a combination thereof, about 0.02 weight % propylparaben, about 0.2 weight % sodium metabisulfite, about 3 weight % stearic acid and about 4 weight % stearyl alcohol.

## Claims

1. A method of making a topical medicated composition, the composition being an emulsion comprising active ingredients consisting of fluocinolone acetonide, hydroquinone and tretinoin, wherein the method comprises the steps of:
(a) preparing an aqueous composition comprising water and at least one hydrophilic compound while heating at a temperature in the range of 75°C to 80°C;
(b) preparing a non-aqueous composition comprising at least two hydrophobic compounds while heating at a temperature in the range of 75°C to 80°C;
(c) combining the aqueous and non-aqueous compositions to form a mixture, wherein steps (a) through (c) are conducted in the absence of an emulsifier;
(d) adding at least one emulsifier to the mixture comprising the aqueous and non-aqueous compositions;
(e) adding tretinoin to the mixture comprising the aqueous and non-aqueous compositions;
(f) adding fluocinolone acetonide to the mixture comprising the aqueous and non-aqueous compositions;
(g) adding hydroquinone to the mixture comprising the aqueous and non-aqueous compositions, wherein the hydroquinone is added after adding the at least one emulsifier; and
(h) homogenizing the mixture to form the emulsion.

2. The method of claim 1, wherein the aqueous composition comprises butylated hydroxytoluene.

3. The method of claim 1, wherein the non-aqueous composition comprises at least two hydrophobic compounds selected from the group consisting of cetyl alcohol, stearic acid, stearyl alcohol, methyl gluceth, methylparaben, propylparaben, and glycerin.

4. The method of claim 1, wherein the non-aqueous composition consists essentially of cetyl alcohol, stearic acid, stearyl alcohol, methyl gluceth, methylparaben, propylparaben, and glycerin.

5. The method of claim 1, wherein step (c) further comprises cooling the mixture to a temperature in the range of 68°C to 72°C.

6. The method of claim 1, wherein the at least one emulsifier is a combination of glycerol stearate and polyethylene glycol stearate.

7. The method of claim 1, further comprising cooling the mixture after adding at least one emulsifier.

8. The method of claim 7, further comprising adding citric acid to the mixture after cooling.

9. The method of claim 8, further comprising cooling the mixture after adding citric acid.

10. The method of claim 1, wherein step (e) is conducted during step (d).

11. The method of claim 10, further comprising cooling the mixture after adding at least one emulsifier and tretinoin.

12. The method of claim 11, further comprising adding citric acid to the mixture after cooling.

13. The method of claim 11, further comprising cooling the mixture after adding citric acid.

14. The method of claim 1, wherein steps (e) and (f) are conducted during step (d).

15. The method of claim 14, further comprising cooling the mixture after adding at least one emulsifier, tretinoin and fluocinolone acetonide.

16. The method of claim 15, further comprising adding citric acid to the mixture after cooling.

17. The method of claim 16, further comprising cooling the mixture after adding citric acid.

18. The method of claim 1, further comprising cooling the mixture before adding the hydroquinone.

19. A topical medicated composition, comprising 0.01 weight% fluocinolone acetonide; 4 weight% hydroquinone; 0.05 weight % tretinoin; 0.04 weight % butylated hydroxy toluene; 4 weight% cetyl alcohol; 0.05 weight% citric acid; 4 weight% glycerin; 5 weight% methyl gluceth; 0.18 weight % methylparaben; 3.5 weight% glyceryl stearate, polyethylene glycol (PEG) stearate or a combination thereof, 0.02 weight% propylparaben, 0.2 weight % sodium metabisulfite, 3 weight% stearic acid and 4 weight % stearyl alcohol.
